# EUROPEAN PATENT SPECIFICATION

(11) **EP 3 361 972 B1**
(45) Date of publication and mention of the grant of the patent: **24.07.2019**
(21) Application number: 16790790.6
(22) Date of filing: 14.10.2016
(51) Int. Cl.: A61B 17/3207, A61B 18/14, A61B 17/3205, A61B 17/00, A61B 18/00, A61N 1/05

(54) **LEAD EXTRACTION DEVICE**
LEITUNGSEXTRAKTIONSGERÄT
DISPOSITIF D'EXTRACTION DE SONDE

(30) Priority: 15.10.2015 US 201562242060 P; 13.10.2016 US 201615292948
(43) Date of publication of application: 22.08.2018
(73) Proprietor: Medtronic Advanced Energy LLC, Minneapolis, MN 55432 (US)
(72) Inventor: BLOOM, Eliot F., Hopkinton, New Hampshire 03229 (US); MARSHALL, Mark T., Forest Lake, Minnesota 55025 (US)
(74) Representative: Dehns
(86) International application number: PCT/US2016/057107
(87) International publication number: WO 2017/066615

(56) References cited:
- US-A- 5 980 515
- US-A- 6 033 402
- US-A1- 2002 049 438
- US-A1- 2011 213 398
- US-A1- 2011 282 373
- US-A1- 2014 081 306
- US-B1- 6 428 539

## Description

### TECHNICAL FIELD

The present invention relates to a device for extracting a medical lead from a patient, and in particular, a combination electrosurgical and mechanical cutting device.

### BACKGROUND

Medical electrical leads are known to provide electrical stimulation therapy to the heart to treat cardiac rhythm disorders and such as atrial fibrillation, tachycardia, and sudden cardiac arrest, which causes upwards of 300,000 deaths annually. Such leads are typically implanted within a chamber of the heart, for example, the right ventricle and are electrically connected to an implantable electrical pulse and/or shock generator known as an implantable cardioverter defibrillator or ICD.

Over time, however, the electrical contact portion of the medical lead, which may be an exposed wire, corrodes or loses contact within the heart such that the effectiveness of the lead becomes reduced and thus the lead must either be replaced or new electrical connections to the heart provided. Removing an electrical lead from the heart, however, is not only expensive and time consuming, but poses numerous risks to the patient, such as injury to cardiac tissue and excessive bleeding.

A medical lead extraction device is disclosed in US 2002/049438.

### SUMMARY

The present invention provides a device as defined in claim 1, for extracting a medical lead from a patient, and in particular, a combination electrosurgical and mechanical cutting device.

A method of use includes sliding a distal end of first shaft of a medical device over the medical lead. The distal end of the first shaft includes a cutting element having a sharp edge. The first shaft is slideably received within a
second shaft. The second shaft is coaxially disposed about the first shaft and includes an electrode at its distal end. Tissue surrounding the medical lead is cut by advancing the distal end of the first shaft distally to the electrode and rotating the cutting element and retracting the distal end of the first shaft within the second shaft and ablating tissue with monopolar radiofrequency energy from the electrode.

In one embodiment, the first shaft defines a major longitudinal axis and the cutting element includes a sharp edge configured to mechanically cut tissue and defining a plurality of slots angled with respect to the major longitudinal axis. The coaxial electrode tapers inward in width as it extends distally. An insulator disposed between the distal end of the shaft and the co-axial electrode is included, the insulator being configured to insulate to the second shaft from the electrode. An actuator coupled to the first shaft is included, the actuator being configured to longitudinally advance and rotate the first shaft independently from the second shaft.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is a side perspective view of an exemplary lead extraction device constructed in accordance with the principles of the present application;
FIG. 2 is a side perspective view of the lead extraction device shown in FIG. 1 with the second shaft slid distally over the first shaft;
FIG. 3 is a side perspective view of another lead extraction device with the second shaft slid distally over the first shaft with a serrated cutting element;
FIG. 4 is a side perspective view of the lead extraction device shown in FIG. 1 coupled to a handle with manual advancement, retraction, and rotation features;
FIG. 5 is a side perspective view of the lead extraction device shown in FIG. 7, with the actuator retracted.
FIG. 6 is a side perspective view of the lead extraction device shown in FIG. 1 coupled to a handle with electrometrical advancement, retraction, and rotation features;
FIG. 7 is a front view inside of a patient showing the device in FIG. 1 being advanced into the heart and cutting through tissue with monopolar radiofrequency ablation energy;
FIG. 8 is a front view inside of a patient showing the device in FIG. 1 being advanced into the heart and mechanically cutting through tissue;
FIG. 9 is a front view inside of a patient showing the device in FIG. 1 being advanced into the right ventricle and mechanically cutting through tissue; and
FIG. 10 is a flow chart illustrating the steps for extracting a medical lead.

### DETAILED DESCRIPTION

As used herein, relational terms, such as "first" and "second," "over" and "under," "front" and "rear," and the like, may be used solely to distinguish one entity or element from another entity or element without necessarily requiring or implying any physical or logical relationship or order between such entities or elements.

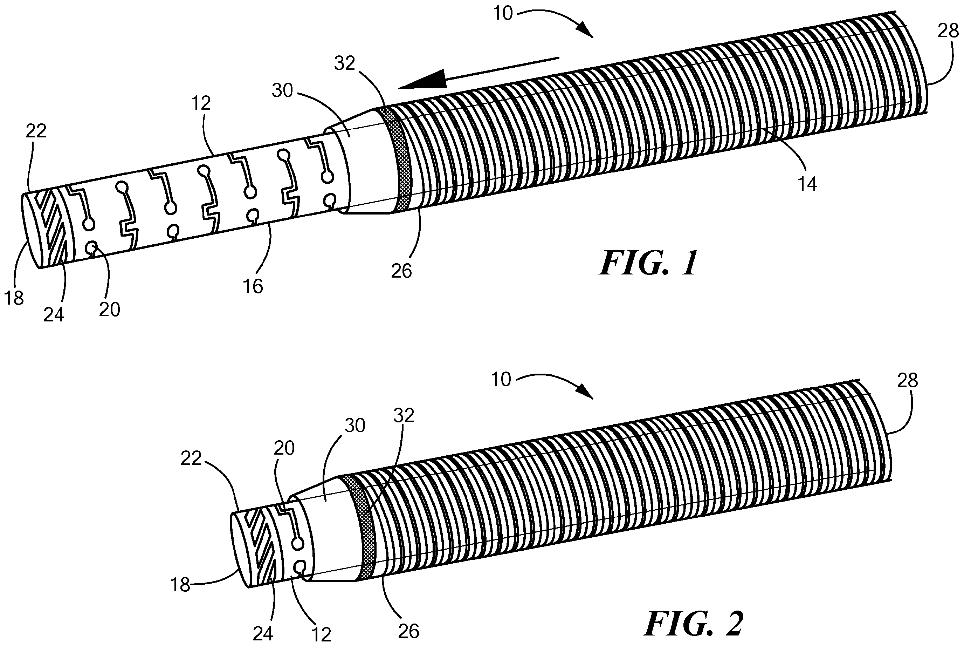

Referring now to the drawings in which like reference designators refer to like elements, there is shown in FIGS. 1-2 an exemplary medical lead extraction device constructed in accordance with the present application and designated generally as "10." The device 10 may include a first shaft 12 defining a proximal portion 14, a distal portion 16, and a lumen 18 there though. The first shaft 12 may a flexible, pushable, and torqueable elongate body such that it is configured to be slid though the vasculature of the patient. The first shaft 12 may be composed of flexible metallic alloy, for example, Nitinol, stainless steel alloys, Tantalum, Titanium and others, and may include a plurality of slits 20 along its surface. For example, the first shaft 12 may include the plurality of slits 20 around at least a portion of its circumference and along at least a portion of its longitudinal length extending from its proximal end to it distal end. In one configuration, the plurality of slits 20 are provided along the entire surface of the first shaft 12 and are laser cut into the shaft to provide a 1:1 torque ratio to the first shaft 12. Each slit in the plurality of slits 20 may define any shape or size and may extend through to the lumen 18. Thus, the shape and size of the slits 20 shown in FIG. 1 is merely exemplary.

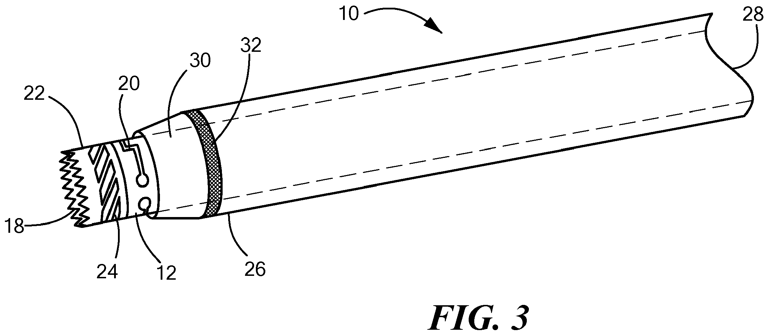

Disposed at the distal end of the distal portion 16 may be a cutting element 22 configured to mechanically cut tissue as it is rotated, or alternatively, as it is pushed. In an exemplary configuration, the cutting element 22 is circumferential and may include a serrated (as shown in FIG. 3) or sharp continuous edge which functions as a saw as the cutting element 22 is rotated. The cutting element 22 may define an outer diameter the same or substantially the same as the outer diameter of the first shaft 12. The cutting element 22 may also include a plurality of slots 24 configured to trap tissue within them. As the cutting element 22 is rotated the tissue lodged within the slots 24, which may be disposed at an oblique angle with respect to the major longitudinal axis, is sliced by a portion of the cutting element 22.

The first shaft 12 may be slideably received within a second shaft 26. In an exemplary configuration the first shaft 12 and the second shaft 26 may be co-axial and/or concentric. The second shaft 26 may define a second lumen 28 sized to slideably receive the first shaft 12. For example, the second lumen 28 may be coated with a lubricious coating such as PTFE to allow the first shaft 12 to slide easily within the second shaft 26. The first shaft 12 and the second shaft 26 may optionally be coupled to handle 29 which may further be in fluid and/or electrical communication with a radiofrequency generator (not shown) to provide irrigation/suction to the device 10, along with electrical power. For example, a fluid such as saline may be pumped through the lumen 18 to irrigate tissue as the device 10 is advanced into the patient. Optionally, the lumen 18 may be in fluid communication with a suction device to suction resected tissue and other material out of the body through the lumen 18.

In an exemplary configuration, as shown in FIGS. 1-2 the second shaft 26 may be coiled as to impart flexibility onto second shaft 26, or may include a coil embedded within the wall of the shaft 26, such as a flat ribbon coil or braided coil. For example, the second shaft 26 may be composed of a conductive material, such as flexible metal or metal alloy, or a non-conductive material such as polyimide, polyamide, combinations of polymers, and the like, which flexes in response to a bending in the first shaft 12. Such a configuration allows the first shaft 12 and the second shaft 26 to move through, for example, the vasculature, and bend and/or flex substantially simultaneously. In other configurations, for example, as shown in FIG. 3, the second shaft may be define a smooth exterior. Disposed at the distal end of the second shaft 26 may be an electrode 30 configured to cut tissue with radiofrequency energy. In an exemplary configuration, the electrode 30 is a ring electrode and is radially disposed around the distal portion 16 of the first shaft 12 and is coupled to a conductor configured to transfer monopolar radiofrequency energy to the electrode 30 from a radiofrequency generator. A return electrode (not shown) may be coupled to the generator and may be disposed on the patient's body, for example, on the back of the patient, to receive energy from the electrode 30. The electrode 30 may further be tapered inward in width as it extends distally. Such a configuration may minimize the risk of tissue being lodged between the first shaft 12 and the second shaft 26. The electrode 30 may optionally be insulated from the remainder of the second shaft 26 by inclusion of an insulator 32 disposed between the distal end of the second shaft 26 and the electrode 30. The insulator 32 may function to electrically insulate the second shaft 26 from the electrode 30 such that radiofrequency energy may be applied solely from the electrode 30 to the tissue.

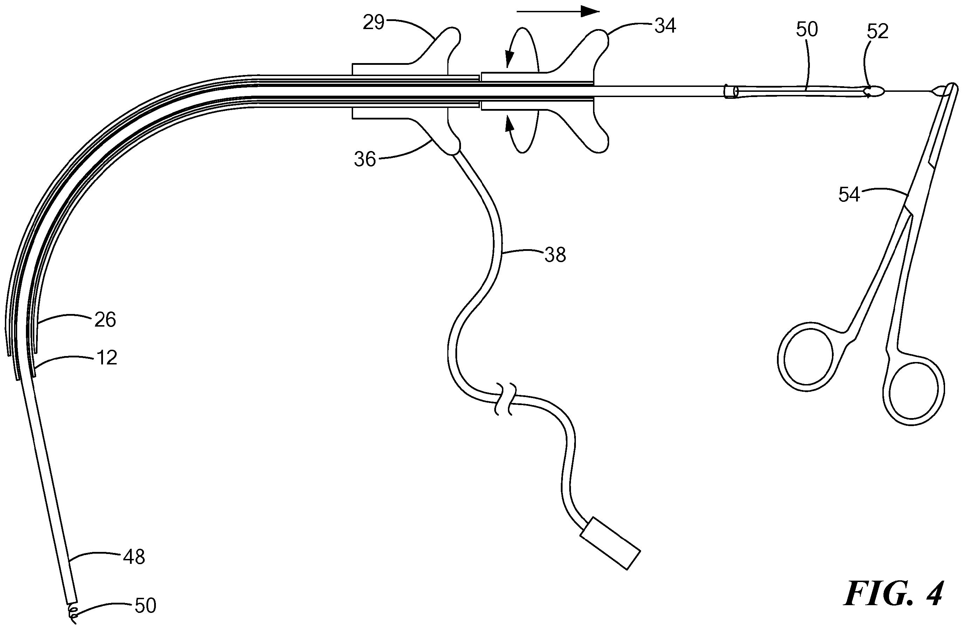

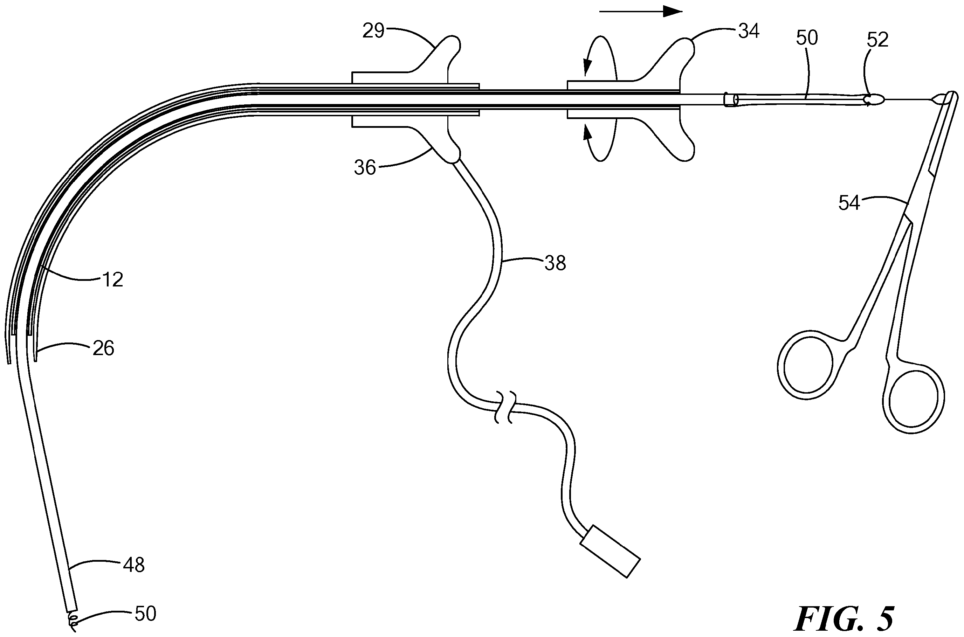

Referring now to FIGS. 4-5, in one configuration of the handle 29, the first shaft 12 is coupled to an actuator 34 which surrounds a portion of the exterior of the first shaft 12. In the embodiment shown in FIG. 4, the actuator 34 is affixed around the circumference of the proximal portion 14 of the first shaft 12. The actuator 34 may have dual functionality in that manually advancing and retracting the actuator 34 advances and retracts the first shaft 12 within the second shaft 26, and manually rotating the actuator 34 rotates the first shaft 12 within the second shaft 26. In an exemplary configuration, the proximal end of the second shaft 26 is positioned such that when the distal end of the actuator 34 is fully advanced, the distal end of the actuator 34 abuts and contacts the proximal end of the shaft 26, which limits the distance the distal portion 16 of first shaft 12 can be advanced out from the distal end of the shaft 26. When the actuator 34 is fully advanced, the cutting element 22 is advanced out of the distal end of the second shaft 26 to a predetermined distance. An electrical coupling 36 may further be coupled to the second shaft 26 to be coupled to a radiofrequency generator to provide power to the electrode 30. In the configuration shown in FIG. 4, the electrical coupling 36 is circumferentially disposed around the second shaft 26 and includes a connector 38 which may be plugged into the radiofrequency generator. The connector 38 may further include a lumen (not shown) that is in fluid communication with the first lumen 18 or the second lumen 28 to provide suction to either of those lumens from a vacuum source (not shown) which may be part of the radiofrequency generator.

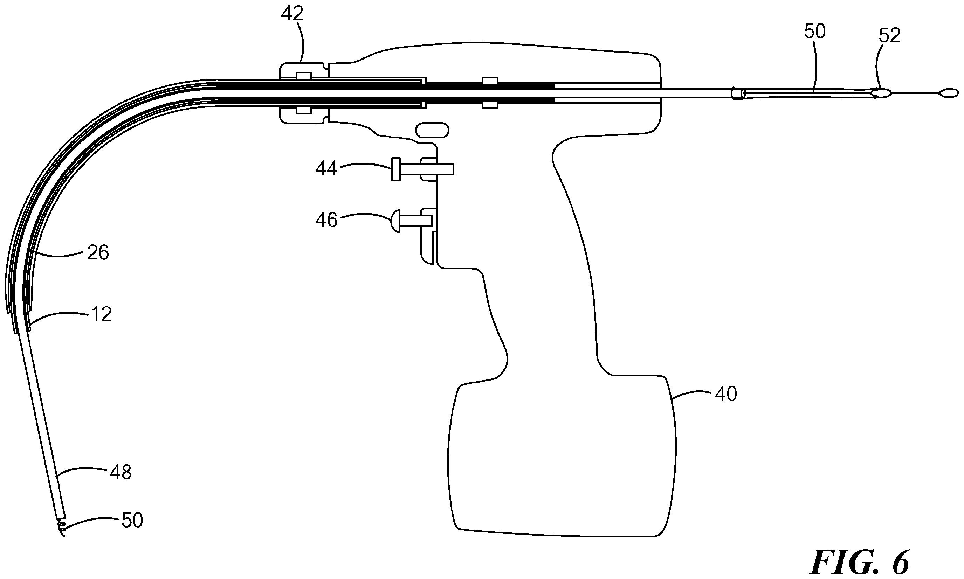

Referring now to FIG. 6, in an alternative configuration, the relative movement and/or rotation of the first shaft 12 and the second shaft 26 may be achieved electro-mechanically or a combination of manual and electromechanic operation. For example, the first shaft 12 and the second shaft 26 may be coupled powered handle 40, which may function to advanced and retract the first shaft 12 with respect to the second shaft 26 or advance and retract the second shaft 26 with respect to the first shaft 12. For example, the first shaft 12 and the second shaft 26 may be coupled to the powered handle 40, which may include a self-contained battery or a plug for coupling to a power a source, by being disposed within a chuck 42 the powered handle 40, which may allow for the first shaft 12 and/or the second shaft 26 to be gripped and released by the powered handle 40. The powered handle 26 may include a first finger switch 44 configured to advance, retract, and/or rotate the first shaft 12 independently of the second shaft 16, and a second finger switch 44 configured to advance, retract, and or rotate the second shaft 26 independently of the first shaft 12. In another configuration, the second shaft 26 may be advanced manually and the first finger switch 44 operates to advance and retract the first shaft 12 and the second finger switch 46 operates to rotate the first shaft 12. The powered handle 40 may include an electrical connection (not shown) such that it couples to a radiofrequency power source.

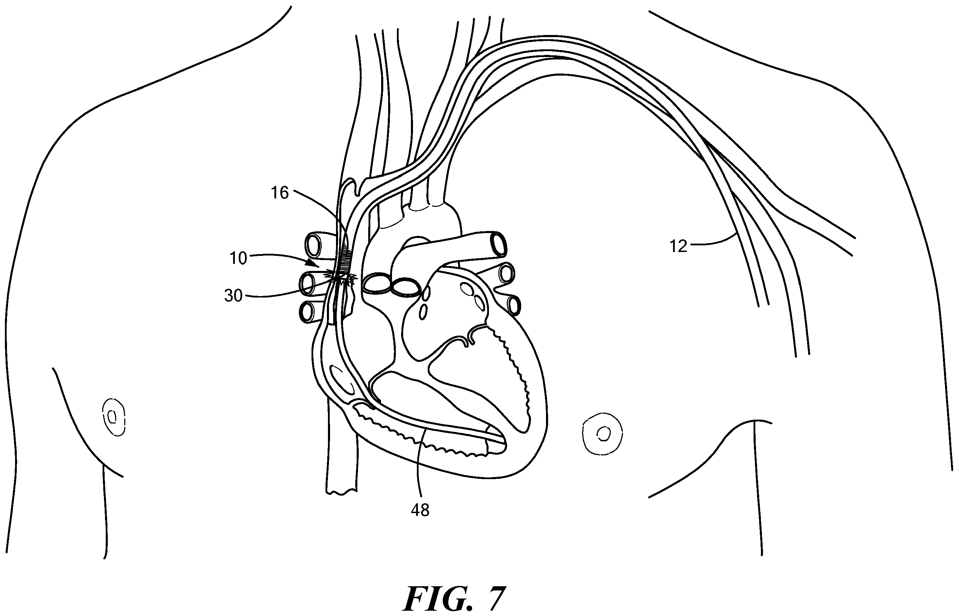

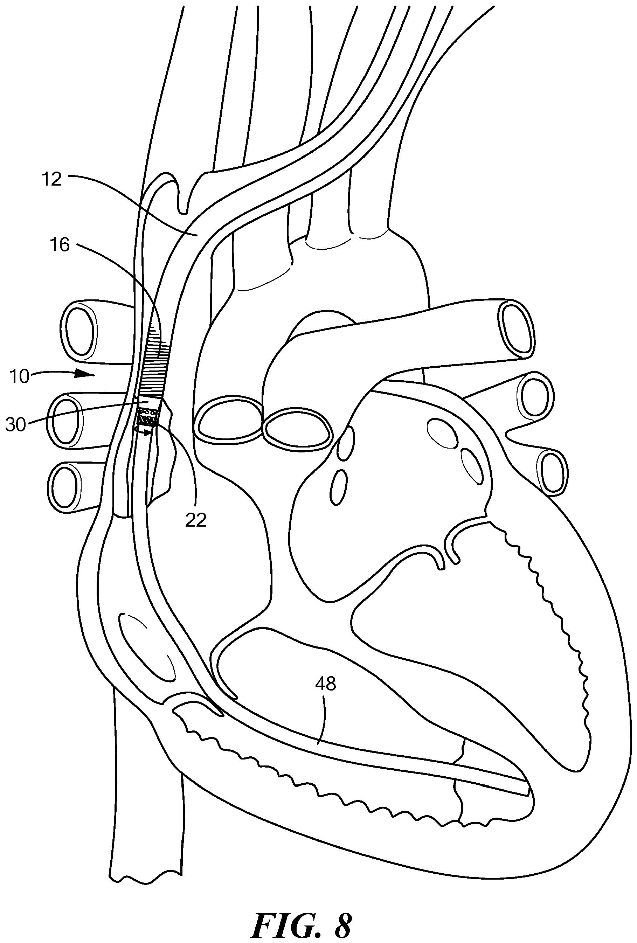

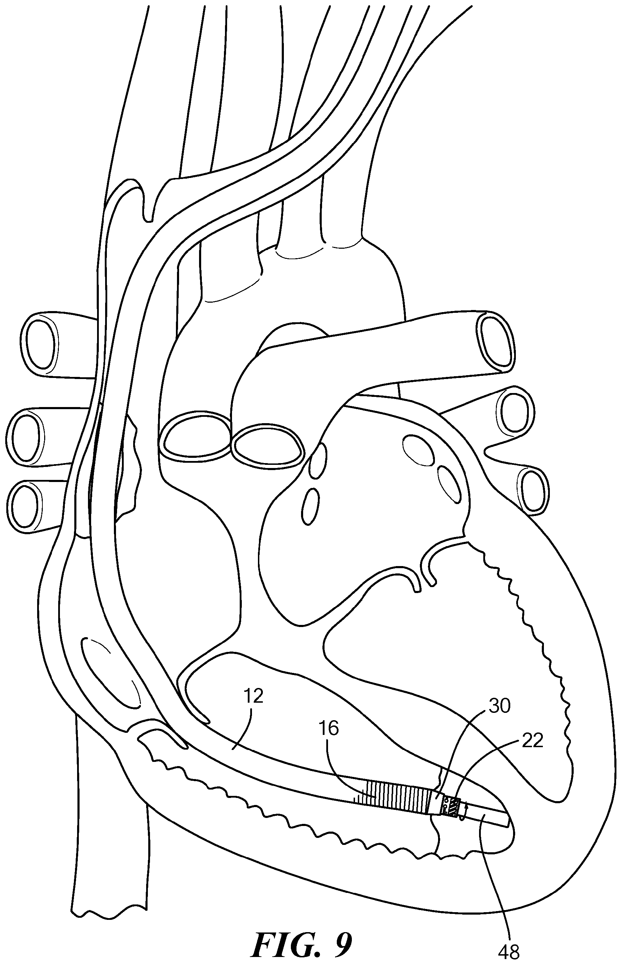

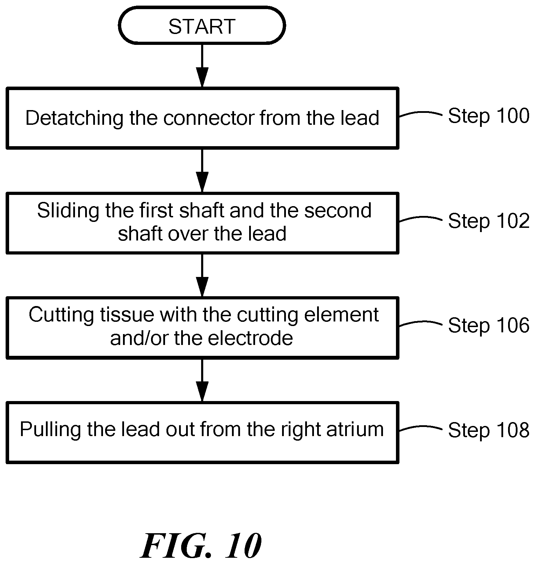

Referring now to FIGS. 7-10, in an exemplary use of the device 10, the user may access, through methods known in the art, the proximal end connector of a medical lead that connects with an implantable cardioverter defibrillator (ICD) or pacemaker, which provides access to lead 48 shown in FIG. 7. The connector (not shown) may be cut off the lead 48 (Step 100) and the device 10 may be slid over the lead 48 (Step 102). For example, as shown in FIGS. 4-5, the lead 48 may be split open and the electrical conductor 50 disposed within may be attached to a locking stylet 52, which is fed through and attached to the lead 48 before the device 10 is advanced over the lead 32, such that when the lead 32 is removed, for example, by forceps 54 (shown in FIGS. 4-5) the device 10 can be slideably removed from the body by sliding over the stylet 52. As the device 10 is advanced through, for example, the brachiocephalic veins and into the superior vena cava, scar tissue and other tissue growth related to the position of the lead 48 within the body may be disposed around and proximate the lead 48. The user may then optionally use the cutting element 22 of the first shaft 12 to mechanically cut tissue around the lead 48 and/or obstructing the pathway of the lead 48 into the heart, or the user may activate the electrode 30 (Step 106). For example, when radiofrequency tissue ablation is desired (FIG. 7), the user may advance the second shaft 26 over the first shaft 12 or independently retract the first shaft 12 into the lumen 28, depending on the position of the first shaft 12 with respect to the second shaft 26, such that the electrode 30 is in contact with tissue to be ablated. When mechanical cutting is desired (FIGS. 8-9) the user may either retract the second shaft 26 or advance the first shaft 12, and rotate the first shaft 12 within the lumen 28 to mechanically cut tissue with the cutting element 22 by rotating the first shaft 12. When the tissue around the distal end of the lead 48 is removed by mechanical or radiofrequency cutting, the user may retract the lead 48 by pulling on the conductor 50 with the forceps 54 within the lumen 18 and remove it from the body (Step 108).

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims. The present invention is defined by the appended claims. The examples, embodiments, or aspects of the present description that do not fall within the scope of said claims are merely provided for illustrative purposes and do not form part of the invention. Furthermore, any surgical, therapeutic, or diagnostic methods presented in the present description are provided for illustrative purposes only and do not form part of the present invention.

## Claims

1. A medical lead extraction device, comprising:
a first shaft (12) defining a proximal end (14), a distal end (16), and a first lumen (18) therebetween sized to receive a medical lead, the first shaft including a cutting element (22) disposed at its distal end, the cutting element including a sharp edge configured to mechanically cut tissue; and
a second shaft (26) defining a proximal end, a distal end, and a second lumen (28) therebetween, the second shaft co-axially surrounding the first shaft and configured to slideably receive the first shaft, the second shaft including a co-axial electrode (30) extending from its distal end and configured to cut tissue with monopolar radiofrequency energy.

2. The device of Claim 1, wherein the electrode (30) tapers in width as it extends distally.

3. The device of Claim 1 or 2, wherein the cutting element (22) has a serrated edge.

4. The device of any preceding Claim, wherein the first shaft (12) is configured to independently rotate within the second lumen (28).

5. The device of any preceding Claim, further including an insulator (32) disposed between the electrode and the distal end of the second shaft, the insulator being configured to insulate the second shaft from the electrode.

6. The device of any preceding Claim, wherein the first shaft includes a plurality of slits (20) along its length, the plurality of slits being configured to impart flexibility onto the first shaft.

7. The device of any preceding Claim, wherein the second shaft at least partially coiled along its length.

8. The device of any preceding Claim, wherein the first shaft is configured to rotate within the second lumen, and wherein the cutting element includes a plurality of slots sized to dislodge tissue as the first shaft rotates.

9. The device of Claim 8, wherein first shaft defines a major longitudinal axis, and wherein the plurality of slots are circumferentially disposed about the cutting element and are angled with respect to the major longitudinal axis.

10. The device of any preceding Claim, further including a handle (29), and wherein the proximal end of the second shaft and the proximal end of the first shaft are coupled to the handle.

11. The device of Claim 10, wherein the handle (29) is configured to electrically couple to an electrosurgical generator configured to deliver radiofrequency ablation energy to the second shaft.

12. The medical lead extraction device of claim 1,
the first shaft defining a major longitudinal axis;
the cutting element defining a plurality of slots angled with respect to the major longitudinal axis; and wherein
the coaxial electrode tapers inward in width as it extends distally; the device further comprising:
an insulator (32) disposed between the distal end of the second shaft and the co-axial electrode, the insulator being configured to insulate to the second shaft from the electrode; and
an actuator (34) coupled to the first shaft, the actuator being configured to longitudinally advance and rotate the first shaft independently from the second shaft.

## Patentansprüche

1. Medizinisches Leitungsextraktionsgerät, umfassend:
einen ersten Schaft (12), der ein proximales Ende (14), ein distales Ende (16) und ein erstes Lumen (18) dazwischen, das so bemessen ist, dass es eine medizinische Leitung aufnimmt, definiert, wobei der erste Schaft ein Schneidelement (22) umfasst, das an seinem distalen Ende angeordnet ist, wobei das Schneidelement eine scharfe Kante umfasst, die konfiguriert ist, um Gewebe mechanisch zu schneiden; und
einen zweiten Schaft (26), der ein proximales Ende, ein distales Ende und ein zweites Lumen (28) dazwischen umfasst, wobei der zweite Schaft den ersten Schaft koaxial umgibt und konfiguriert ist, um den ersten Schaft verschiebbar aufzunehmen, wobei der zweite Schaft eine koaxiale Elektrode (30) umfasst, die von seinem distalen Ende aus verläuft und konfiguriert ist, um Gewebe mit einpoliger Hochfrequenzenergie zu schneiden.

2. Gerät nach Anspruch 1, wobei die Elektrode (30) sich in der Breite verjüngt, während sie sich distal erstreckt.

3. Gerät nach Anspruch 1 oder 2, wobei das Schneidelement (22) eine gezackte Kante aufweist.

4. Gerät nach einem der vorstehenden Ansprüche, wobei der erste Schaft (12) konfiguriert ist, um sich unabhängig innerhalb des zweiten Lumens (28) zu drehen.

5. Gerät nach einem der vorstehenden Ansprüche, ferner umfassend einen Isolator (32), der zwischen der Elektrode und dem distalen Ende des zweiten Schafts angeordnet ist, wobei der Isolator konfiguriert ist, um den zweiten Schaft von der Elektrode zu isolieren.

6. Gerät nach einem der vorstehenden Ansprüche, wobei der erste Schaft eine Vielzahl von Spalten (20) entlang seiner Länge umfasst, wobei die Vielzahl von Spalten konfiguriert ist, um dem ersten Schaft Flexibilität zu verleihen.

7. Gerät nach einem der vorstehenden Ansprüche, wobei der zweite Schaft zumindest teilweise entlang seiner Länge gewunden ist.

8. Gerät nach einem der vorstehenden Ansprüche, wobei der erste Schaft konfiguriert ist, um sich innerhalb des zweiten Lumens zu drehen, und wobei das Schneidelement eine Vielzahl von Schlitzen umfasst, die so bemessen sind, dass sie Gewebe ablösen, wenn sich der erste Schaft dreht.

9. Gerät nach Anspruch 8, wobei der erste Schaft eine Hauptlängsachse definiert und wobei die Vielzahl von Schlitzen in Umfangsrichtung um das Schneidelement angeordnet ist und in Bezug auf die Hauptlängsachse abgewinkelt ist.

10. Gerät nach einem der vorstehenden Ansprüche, ferner umfassend einen Griff (29), und wobei das proximale Ende des zweiten Schafts und das proximale Ende des ersten Schafts mit dem Griff verbunden sind.

11. Gerät nach Anspruch 10, wobei der Griff (29) konfiguriert ist, um sich elektrisch mit einem elektrochirurgischen Generator zu verbinden, der konfiguriert ist, um dem zweiten Schaft Hochfrequenzablationsenergie bereitzustellen.

12. Medizinisches Leitungsextraktionsgerät nach Anspruch 1, wobei der erste Schaft eine Hauptlängsachse definiert;
wobei das Schneidelement eine Vielzahl von Schlitzen definiert, die in Bezug auf die Hauptlängsachse abgewinkelt sind; und wobei die koaxiale Elektrode sich nach innen in der Breite verjüngt, während sie sich distal erstreckt; wobei das Gerät ferner Folgendes umfasst:
einen Isolator (32), der zwischen dem distalen Ende des zweiten Schafts und der koaxialen Elektrode angeordnet ist, wobei der Isolator konfiguriert ist, um den zweiten Schaft von der Elektrode zu isolieren; und
ein Antriebselement (34), das mit dem ersten Schaft verbunden ist, wobei das Antriebselement konfiguriert ist, um den ersten Schaft in Längsrichtung unabhängig vom zweiten Schaft vorzuschieben und zu drehen.

## Revendications

1. Dispositif d'extraction de dérivation médicale, comprenant :
une première tige (12) définissant une extrémité proximale (14), une extrémité distale (16) et une première lumière (18) entre elles dimensionnée pour recevoir une dérivation médicale, la première tige comportant un élément de coupe (22) disposé à son extrémité distale, l'élément de coupe comportant un bord acéré configuré pour couper mécaniquement un tissu ; et
une seconde tige (26) définissant une extrémité proximale, une extrémité distale et une seconde lumière (28) entre elles, la seconde tige entourant coaxialement la première tige et configurée pour recevoir de manière coulissante la première tige, la seconde tige comportant une électrode coaxiale (30) s'étendant à partir de son extrémité distale et configurée pour couper un tissu à l'aide d'une énergie radiofréquence monopolaire.

2. Dispositif selon la revendication 1, dans lequel l'électrode (30) s'effile en largeur au fur et à mesure qu'elle s'étend distalement.

3. Dispositif selon la revendication 1 ou 2, dans lequel l'élément de coupe (22) possède un bord dentelé.

4. Dispositif selon une quelconque revendication précédente, dans lequel la première tige (12) est configurée pour tourner indépendamment de la seconde lumière (28).

5. Dispositif selon une quelconque revendication précédente, comportant en outre un isolant (32) disposé entre l'électrode et l'extrémité distale de la seconde tige, l'isolant étant configuré pour isoler la seconde tige de l'électrode.

6. Dispositif selon une quelconque revendication précédente, dans lequel la première tige comporte une pluralité de fentes (20) le long de sa longueur, la pluralité de fentes étant configurées pour conférer de la souplesse à la première tige.

7. Dispositif selon une quelconque revendication précédente, dans lequel la seconde tige est au moins partiellement enroulée le long de sa longueur.

8. Dispositif selon une quelconque revendication précédente, dans lequel la première tige est configurée pour tourner à l'intérieur de la seconde lumière, et dans lequel l'élément de coupe comporte une pluralité de fentes dimensionnées pour déloger du tissu alors que la première tige tourne.

9. Dispositif selon la revendication 8, dans lequel la première tige définit un axe longitudinal principal, et dans lequel la pluralité de fentes sont disposées circonférentiellement autour de l'élément de coupe et sont inclinées par rapport à l'axe longitudinal principal.

10. Dispositif selon une quelconque revendication précédente, comportant en outre une poignée (29), et dans lequel l'extrémité proximale de la seconde tige et l'extrémité proximale de la première tige sont couplées à la poignée.

11. Dispositif selon la revendication 10, dans lequel la poignée (29) est configurée pour se coupler électriquement à un générateur électrochirurgical configuré pour délivrer une énergie d'ablation radiofréquence à la seconde tige.

12. Dispositif d'extraction de dérivation médicale selon la revendication 1,
la première tige définissant un axe longitudinal principal ;
l'élément de coupe définissant une pluralité de fentes inclinées par rapport à l'axe longitudinal principal ; et dans lequel
l'électrode coaxiale s'effile vers l'intérieur en largeur au fur et à mesure qu'elle s'étend distalement ; le dispositif comprenant en outre :
un isolant (32) disposé entre l'extrémité distale de la seconde tige et l'électrode coaxiale, l'isolant étant configuré pour isoler la seconde tige de l'électrode ; et
un actionneur (34) couplé à la première tige, l'actionneur étant configuré pour faire avancer longitudinalement et faire tourner la première tige indépendamment de la seconde tige.
